# EUROPEAN PATENT SPECIFICATION

(11) **EP 1 768 624 B1**
(45) Date of publication and mention of the grant of the patent: **23.12.2009**
(21) Application number: 05760747.5
(22) Date of filing: 07.07.2005
(51) Int. Cl.: A61F 5/445, A61F 5/451

(54) **CONTAINER FOR COLLECTING EXCRETIONS, DRAINING COLLECTIONS, PURGING OSTOMIES OR THE LIKE**
BEHÄLTER ZUR SAMMLUNG VON EXKRETEN, DRAINAGE, ZUR SPÜLUNG VON OSTOMIEN ODER DERGLEICHEN
CONTENANT POUR RECUEILLIR LES EXCRETIONS, EVACUER LES SUBSTANCES RECUEILLIES, PURGER LES STOMIES OU SIMILAIRE

(30) Priority: 08.07.2004 IT MO20040177
(43) Date of publication of application: 04.04.2007
(73) Proprietor: Palmieri, Beniamino, I-41100 Modena (IT)
(72) Inventor: Palmieri, Beniamino, I-41100 Modena (IT)
(74) Representative: Modiano, Micaela Nadia
(86) International application number: PCT/EP2005/053250
(87) International publication number: WO 2006/005717

(56) References cited:
- EP-A- 0 890 349
- WO-A-97/01316
- GB-A- 2 268 882
- US-A- 4 197 849
- US-A- 5 263 947
- US-A1- 2003 045 843

## Description

### Technical Field

The present invention relates to a container for collecting excretions, draining collections, purging ostomies or the like.

### Background Art

In the medical and surgical field, various containers are known which are intended exclusively to collect excretions, urine or feces, or to drain collections or purge ostomies.

For the collection of feces, in addition to disposable diapers or so-called bedpans, containers are known which are substantially constituted by bags which are closed along their perimetric edges and on one face of which there is a circular opening in which the rim is associated with an adhesive element temporarily protected by a removable film and the diameter is such as to surround only the sphincter.

After removing the film, the rim of the opening is made to adhere to the skin that surrounds the sphincter.

In order to apply these containers, instruments are known which are substantially constituted by a bell-shaped retractor, which accommodates internally a disk-like support so that it can slide axially, said support being intended to support the container and being associated with the end of a stem arranged, so that it can slide axially, in a sleeve that lies to the rear of the retractor and ends at the opposite end with a handle.

However, these known containers are not free from drawbacks, including the fact that they do not ensure perfect adhesion of the rim of the opening to the skin that surrounds the sphincter; this rim, as a consequence of a successive contractions and expansions of the sphincter region, in fact tends to come unstuck locally, reducing the seal of the container, causing unwanted external leakage and forming pockets in which the feces collect partly, contaminating the perineal region and thus facilitating the onset of infections, macerations and sores.

Known instruments for applying these containers also have drawbacks, including the fact that they are awkward to use and that they do not adapt to the various anatomical configurations of different patients and therefore do not allow to make the rim of the opening adhere completely to the respective perineal regions.

In order to collect urine, catheters are instead known which are connected to a bag or, for application to male patients, condom-like sheaths are known which lead into a duct which discharges into a bag.

Catheters and sheaths, however, are awkward and unpleasant to use, since in any case they are invasive; moreover, sheaths, by adhering elastically to the penis, cause unpleasant constriction sensations.

Finally, appropriately provided bags are known for purging ostomies, such as colostomies, but they require large flat regions in order to ensure the adhesion of their inlet to the skin and can be applied only to apertures that are not subjected to successive contractions and relaxations, from which they would otherwise detach.

Therefore, the use of these bags is inconveniently limited only to the purging of ostomies.

It is therefore necessary to have different containers depending on the nature of the material to be collected and on the orifice from which it is to be evacuated, and this accordingly entails high costs for the manufacture and management of various store reserves and waste of material.

Incontinent aids, in the form of collecting bags, are known from documents US-4197849 and US-5263947.

### Disclosure of the Intention

The aim of the present invention is to eliminate the drawbacks noted above, by providing a container for collecting excretions, draining collections, purging ostomies or the like which is versatile and flexible in use, hygienic and safe, ensures tightness against leaks and avoids contamination of the skin of patients, preserving them from the risk of infections, macerations and sores.

Further obj ects of the present invention are to provide a container that is simple, quick and easy to apply, allows to protect medical staff and health workers assigned to its use from the risk of infections or contaminations, and allows them full freedom in performing local procedures on the involved orifice, such as for example the application of medicines, ointments or the like, maneuvers with surgical or diagnostic instruments, washing or others.

An instrument for applying the container that is easy to use and flexible and adaptable to the anatomical configuration of different patients, allowing in any case perfect application of the container is also described. The instrument is described as an example and does not form part of the matter for which the protection is sought.

Within this aim, an object of the present invention is to provide a container that is simple, relatively easy to provide in practice, safe in use, effective in operation, and has a relatively low cost.

This aim and these and other objects that will become better apparent hereinafter are achieved by the present container for collecting excretions, draining collections, purging ostomies or the like, according to the invention, that has the features set forth in claim 1.

This aim and these objects are also achieved by a device for applying the container to the perineal region, **characterized in that** it comprises two blades, between which spring means are interposed which are suitable to keep them mutually close and are provided with a handle.

### Brief description of the Drawings

Further characteristics and advantages of the present invention will become better apparent from the following detailed description of a preferred but not exclusive embodiment of a container for collecting excretions, draining collections, purging ostomies or the like and of the corresponding applicator, illustrated by way of non-limiting example in the accompanying drawings, wherein:

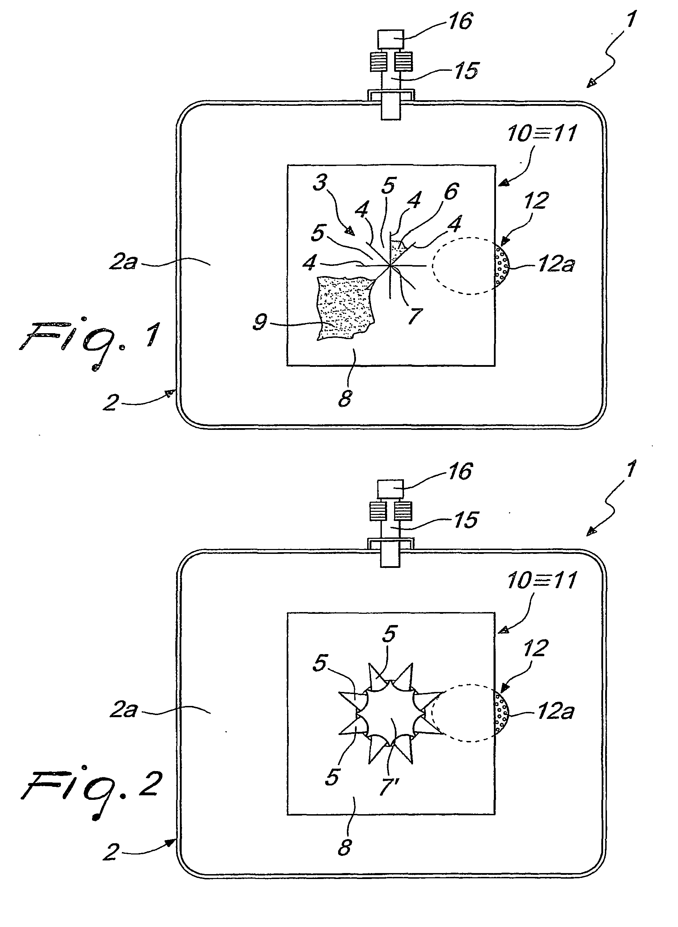

Figure 1 is a schematic and partially cutout plan view of a container according to the invention;
Figure 2 is a schematic plan view of the container according to the invention, with the sectors raised so as to form the passage opening;

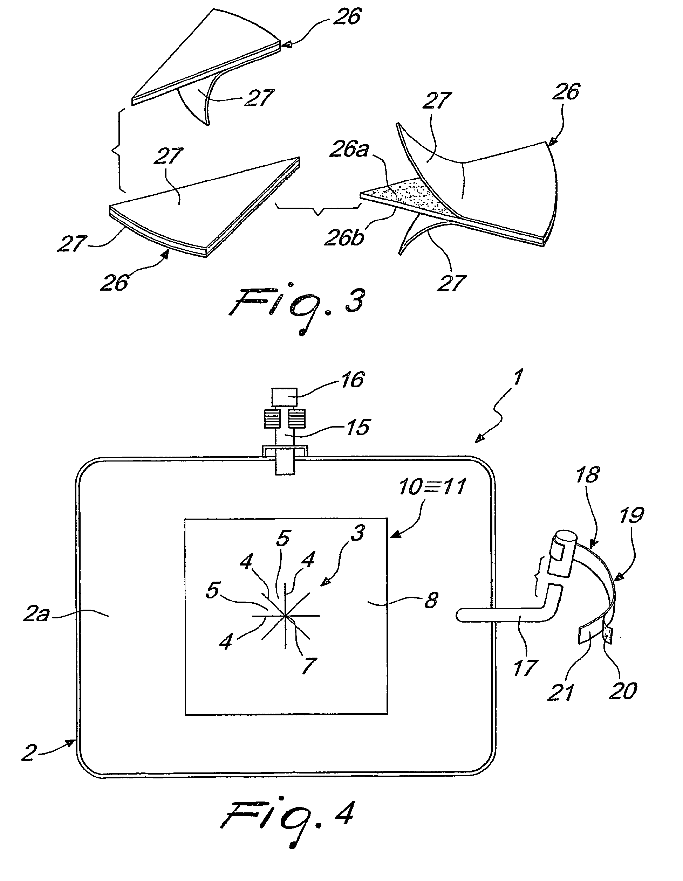

Figure 3 is a schematic perspective view of the tabs for applying the container according to the invention;
Figure 4 is a schematic plan view of a first alternative embodiment of the container according to the invention;
Figure 5 is a schematic plan view of a second alternative embodiment of the container according to the invention;
Figure 6 is a schematic front sectional view of an applicator given as example;

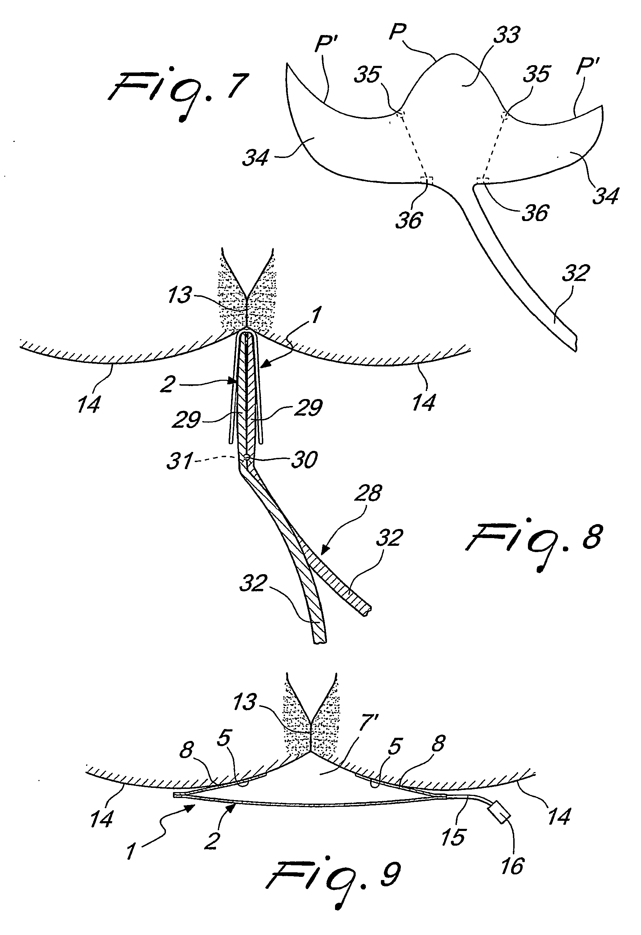

Figure 7 is a schematic side view of the applicator;
Figure 8 is a schematic view of the step for applying the container according to the invention to the perineal region of a patient in order to collect feces;
Figure 9 is a schematic view of the container according to the invention applied to the perineal region of a patient.

### Ways of currying out the Invention

With reference to the figures, the reference numeral 1 generally designates a container for collecting excretions, draining collections, purging ostomies or the like.

The container 1 is applied in the medical and surgical field to collect feces and/or urine of immobilized, disabled or incontinent patients, for draining collections from infected cavities, for example in thoracic surgery, in cleaning surgical wounds, such as for example in liver transplants, for hepatic abscesses or abscesses of the abdominal wall that have fistulized externally, for rupturing aneurysms (rexin), in order to recover the blood to be reinfused by autotransfusion, for draining hydrothoraces and chylothoraces, and the like.

Further, the container 1 can be used for wounds complicated by colliquating and suppurating hematomas, for traumatic wounds and lacerated wounds that cannot be sutured, to isolate a surgical incision, whether contaminated or not, and to evacuate necrotic or serohematic or purulent material from cavities, such as for example are from the vaginal cavity, in order to analyze the quality and quantity of the material produced following surgical procedures, infectious processes, hemorrhagic or colliquative processes, without the use of sanitary towels or diapers.

The container 1 comprises a bag 2, which is closed along its peripheral edges and on at least one face 2a of which there is a radial arrangement 3 of slits or cuts 4, which form a plurality of sectors 5, which are mutually independent and each of which is associated, at its surface directed toward the outside of the bag 2, with a respective adhesive element 6, which is intended to adhere to a portion of the skin of a patient that surrounds the exit orifice of the excretion to be collected, the collection to be drained, or the material to be purged.

The radial arrangement 3 forms a hole 7; when the sectors 5 adhere to the skin of the patient, lifting from the plane of arrangement of the face 2a, the hole 7 expands into an opening 7' for the passage of the excretions, collections or material.

A rim 8 is formed around the radial arrangement 3 and is associated with a respective adhesive element 9, which is suitable to adhere to the peripheral skin of the affected orifice.

The adhesive elements 6 and 9, respectively of the sectors 5 and the rim 8, are covered by a respective protective film 10 and 11, which is detachably associated therewith; advantageously, the two films 10 and 11 are formed in a single sheet.

One face of a flap 12 can be associated on a fraction of the adhesive element 6 and/or 9, and its opposite face 12a, made of non-stick and advantageously porous and breathable material, is designed to cover a portion of the skin of the patient, such as for example the vulvar region, protecting it from sticking to the adhesive element 6 and/or 9.

If the container 1 is intended to collect human feces, the hole 7 is arranged proximate to the anus 13 and the sectors 5 are made to adhere to the skin of the glutei 14 that surrounds the anus 13; in this case, the sectors 5 have a height that is substantially equal to 3.5-4 cm, the opening 7' having an average diameter of approximately 7 cm.

Further, the container 1 comprises a duct 15 for accessing the bag 2, in which one end is associated with the bag 2 and the opposite end is associated with removable closure means, such as for example a screw-on cap 16. Conveniently, it is possible to associate with the duct 15 a one-way valve, not shown as it is of a know type, isolating the operating environment enclosed by the bag 2 and avoiding its contamination from the outside.

It is possible to insert through the duct 15 cannulas for the administration of enemas or washing solutions, syringes or atomizers for applying or spraying disinfectants, medications, ointments or others, surgical instruments such as for example laparoscopes or the like in order to operate for example on polyps, hemorrhoids or condylomas, diagnostic instruments or others.

If the container 1 is intended also to collect urine, it comprises a tubular element 17, in which one end is associated with the bag 2 and the opposite end is suitable to be associated with the urethral orifice and provided with means 18 for adhering to the penis or vulva.

If the container 1 is intended to be applied to a male patient, the adhesion means 18 comprise a tape 19 provided with an adhesive element 20 on one side, said element being covered by a respective protective film 21 of the detachable type and being suitable to be wrapped around the glans and/or penis.

If the container 1 is intended to be applied to a female patient, a collection cup 22 is associated with the free end of the tubular element 17 and is intended to be placed against the vulva, and the adhesion means 18 comprise an adhesive element 23, which is distributed along the upper perimetric rim 24 of the cup 22 and is covered by a respective removable protective film 25.

Conveniently, a spongy element, not shown, suitable to absorb urine and make it percolate into the tubular element 17, can be accommodated inside the cup 22.

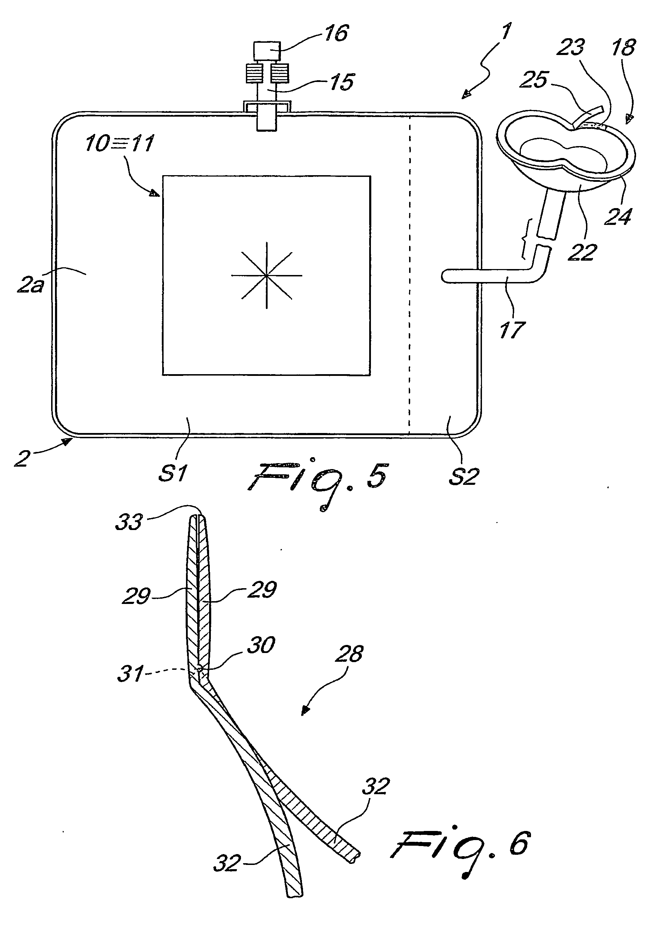

If the container 1 is intended to collect feces and urine, the bag 2 can be divided advantageously into two separate compartments S1 and S2 (Figure 5), in which one compartment S1 is designed to collect feces and has the radial arrangement 3 of cuts 4 formed thereat, the hole 7 of said radial arrangement being suitable to be arranged at the anus 13, and the other compartment S2 is designed to collect urine and has the tubular element 17 associated thereat.

It is possible to insert in the bag 2 material which adsorbs gases and/or coagulates and/or thickens the collected fluids, or it is possible to aerosolize inside it drugs or other compounds or inject gases.

To facilitate the application of the container 1, the use is provided of a plurality of double-adhesive tabs 26 made of hydrocolloidal resinous material, polyurethane material or the like, having various thicknesses, which are suitable to be interposed between the sectors 5, of which they substantially duplicate the shape and dimensions, and/or the rim 8 and the skin or between the adhesion means 18 and the penis or vulva.

In particular, the tabs 26 are substantially shaped like a triangle, advantageously an isosceles triangle.

A respective removable protective film 27 is applied to each one of the opposite adhesive faces 26a and 26b of each double-adhesive tab 26.

The tabs 26 are applied to the perianal skin or in the vicinity of another orifice before applying the bag 2 and increase the adhesion and seal of the sectors 5 of the bag 2.

A preferred embodiment uses curved or arc-like double-adhesive rods or bars, not shown, in order to increase the adhesion of the sectors 5 to the skin and as a replacement of the tabs 26.

Advantageously, the bars or rods have a length comprised between 0.5 and 1 cm, a width comprised between 0.2 and 4 cm, and a thickness comprised between 0.2 and 0.4 cm.

Such bars or rods are applied to the perianal skin or to the skin that surrounds another orifice and are arranged in one or more concentric rows with their concavity directed toward the anus or other orifice, and the sectors 5 of the bag 2 are then applied onto them. Conveniently, the bars or rods are replaced each time the bag 2 is changed.

In order to apply the container 1 at the perineal region, an applicator 28 is used which comprises two blades 29, which are mutually pivoted about a pivot 30 and between which spring means 31 are interposed which are suitable to keep them mutually close; said blades are provided with a handle 32.

Each blade 29, viewed laterally, comprises a central portion 33, which has a substantially convex profile P and is intended to be arranged at the anus 13; two wings 34 extend in opposite directions from said central portion, have a substantially concave profile P' and are designed to surround the glutei.

In order to be able to adapt the applicator 28 to the different anatomical configurations of different patients, the wings 34 can be pivoted to the central portion 33 about respective pivots 35, means 36 for fixing their relative position being provided.

Conveniently, the profile P of the central portion 33 protrudes at least partially with respect to the profile P' of the wings 34 and the handle 32 is inclined with respect to the longitudinal axis of the central portion 33 or with respect to the plane of arrangement of the two blades 29.

The bag 2 is made of impermeable polymeric material, in particular polyuethane material; the adhesive elements and the double-adhesive tabs 26 are polyurethane-based.

In order to increase the adhesiveness of the adhesive elements 6 and 9 of the bag 2 and of the tabs 26 or of the bars or rods, it is possible to modify their electrostatic charge; in particular, since the skin is characterized by a negative electrostatic charge, it is possible to modify the adhesive of these components with a positive electrostatic charge, for example by dispersing inside it positively charged particles, magnetite or titanium. In this manner, the adhesion to the skin of the adhesive elements 6 and 9 and of the tabs 26 and of the rods or bars is increased by their mutual electrostatic attraction.

Particularly but not exclusively with reference to the application of the container 1 in the perineal region to collect feces, the operation of the invention is as follows.

After removing the films 10 and 11 that cover the adhesive elements 6 and 9 of the sectors 5 and of the rim 8, the bag 2 is rested against the applicator 28 so that the central axis of said bag lies substantially at the end of the blades 29 and half of the bag 2 falls onto the opposite sides of said blades, and the hole 7 is substantially at the apex of the profile P of their central portion 33.

After parting the glutei 14 of the patient, the applicator 28 is inserted, with the bag 2 rested thereon, between said glutei, centering the anus 13 with the central portion 33, and by applying pressure to the handle 32, overcoming the resisting force of the spring means 31, the two blades 29 are moved mutually apart, pressing the sectors 5, and possibly part of the rim 8, against the skin of the patient, to which they adhere.

In case of hypotonus and weakness of the sphincter, it is possible to insert the sectors 5, having an appropriate height, within the anal orifice so as to line its internal walls and skim its ring, facilitating, during expulsion, the entry of the cylinder of feces into the bag 2.

Before applying the container 1, it is possible to arrange around the anus 13 the double-adhesive tabs 26 and/or spray the skin with a fluid adhesive, based for example on gum arabic, so as to improve the adhesion of the sectors 5 and/or of the rim 8 to said skin.

If the tabs 26 are also used, said tabs, having an appropriate height, can be arranged so that their base lies proximate to the rim of the anal orifice and the opposite apex lies so as to adhere to the internal wall of the anal orifice, so as to constitute a guide and a seal for the expulsion of the cylinder of feces into the bag 2, minimizing the possible contamination of the perianal skin.

Once the collection of the material has ended, it is sufficient to detach the bag 2 and fold back the adhesive elements 6 and 9 so as to seal the content of the bag 2 with respect to the external environment without contaminations and without exhalations.

The container according to the invention is also used in surgical procedures and anorectal endoscopic maneuvers, acting both as a device for collecting the material produced in the operated area, to be subjected to possible analysis, without external contaminations and without releasing any exhalations, and as a medication as a replacement of conventional gauzes; the duct for accessing the bag in fact allows to apply locally medications, drugs or others.

The container according to the invention is further used in all cases in which it is necessary to collect in a sterile manner material to be subjected to analysis (bacterial analyses, mycotic analyses, antibiograms, biochemical analyses, chemical-physical analyses, etc), such as for example analysis of occult blood in feces, protecting the collected material against external contaminations and therefore without affecting the results of the analyses performed thereon.

The container according to the invention can be used to collect material from all the cavities connected to movable surfaces or surfaces provided with folds, such as for example the cavities formed in the cervical, mastoid, pelvic, axillary or similar cavities, for collecting for example material from fistulas.

In practice it has been found that the described invention achieves the intended aim and obj ects.

The invention thus conceived is susceptible of numerous modifications and variations, all of which are within the scope of the appended claims.

All the details may further be replaced with other technically equivalent ones coming within the scope of the claims.

In practice, the materials used, as well as the shapes and the dimensions, may be any according to requirements without thereby abandoning the scope of the protection of the appended claims.

## Claims

1. A container for collecting excretions, draining collections, purging ostomies or the like, comprising a bag (2) which is closed along its peripheral edges and has on at least one face thereof a radial arrangement (3) of slits (4) which form a plurality of independent sectors (5), adhesive elements (6, 9, 26) are further provided intended to adhere to a portion of the skin of a patient which surrounds the exit orifice of the excretion, collection or material to be purged, the hole (7), which constitutes the center of said radial arrangement (3), being suitable to expand into an opening (7') for the passage of said excretion, collection or material, **characterized in that** each one of said independent sectors (5) is associated with each one of said adhesive elements (6, 26) at its surface directed to the outside of the bag (2), the bag (2) being made of polyurethane material and the adhesive elements (6, 9, 26) being polyurethane-based.

2. The container according to claim 1, **characterized in that** it comprises a rim (8) which is formed so as to surround said radial arrangement (3) and is associated with a respective adhesive element (9) and is suitable to adhere to the skin of said patient that is located peripherally with respect to said orifice.

3. The container according to one or more of the preceding claims, **characterized in that** it comprises at least one protective film (10, 11), which is associated detachably with the adhesive element (6, 9) of said sectors (5) and/or said rim (8).

4. The container according to one or more of the preceding claims, **characterized in that** it comprises a flap (12) provided with a side which is rigidly associated with a fraction of the adhesive element (6, 9) of said sectors (5) and/or of said rim (8) and an opposite side made of non-stick material and intended to preserve a portion of the skin of said patient from adhesive bonding with said adhesive element (6, 9).

5. The container according to one or more of the preceding claims, **characterized in that** said sectors (5) have a height that can vary between 0.5 cm and 7 cm, preferably equal to 3.5 cm.

6. The container according to one or more of the preceding claims, **characterized in that** it comprises at least one duct (15) for access to said bag (2), in which one end is associated with said bag (2) and the opposite end is associated with removable closure means.

7. The container according to claim 6, **characterized in that** it comprises a one-way valve associated with said duct (15).

8. The container according to one or more of the preceding claims, **characterized in that** it comprises a tubular element (17) in which one end is associated with said bag (2) and the opposite end is suitable to be associated with the urethral orifice and is provided with means (18) for adhesion to the penis or vulva.

9. The container according to claim 8, **characterized in that** said adhesion means (18) comprise a tape (19) which is provided, on one side, with an adhesive element (20) and is suitable to be wrapped around the penis.

10. The container according to one of the claims 7-9, **characterized in that** said opposite end of the tubular element (17) is associated with a collecting cup (22), which is intended to be placed against the vulva, said adhesion means (18) comprising an adhesive element (23), which is distributed along the upper perimetric rim (24) of said cup (22).

11. The container according to claim 10, **characterized in that** it comprises a spongy element accommodated within said cup (22).

12. The container according to one or more of the preceding claims, **characterized in that** it comprises a protective film (10, 11, 21) which is detachably associated with the adhesive element (6, 9) of said tape (19) or of said rim (8).

13. The container according to one or more of the preceding claims, **characterized in that** said bag (2) comprises at least two separate compartments (51, 52), one of said compartments (51) being intended to collect the feces, said radial arrangement (3) of slits (4) being formed thereat, its hole (7) being suitable to be arranged at the anus (1), the other compartment (53) being intended to collect urine and said tubular element (17) being associated thereat.

14. The container according to one or more of the preceding claims, **characterized in that** it comprises adsorbent and/or coagulating and/or thickening material accommodated within said bag (2).

15. The container according to one or more of the preceding claims, **characterized in that** it comprises at least one double-adhesive tab (26), which is suitable to be interposed between said sectors (5) and/or said rim (8) and said skin or between said adhesion means (18) and said penis or vulva.

16. The container according to claim 15, **characterized in that** said tab (26) comprises a protective film (27), which is detachably associated with each one of its opposite sides (26a, 26b).

## Patentansprüche

1. Ein Behälter zur Sammlung von Exkreten, Drainage, zur Spülung von Ostomien oder dergleichen, mit einem Beutel (2), der entlang seiner peripheren Kanten geschlossen ist und an einer von seinen Seiten eine radiale Anordnung (3) an Schlitzen (4) aufweist, die eine Mehrzahl unabhängiger Sektoren (5) bilden, wobei des Weiteren adhäsive Elemente (6, 9, 26) vorgesehen sind, die ausgebildet sind, um an einem Teil der Haut eines Patienten zu kleben, die die Ausgangs-Öffnung der Exkretion, der Sammlung oder des zu entleerenden Materials umgibt, wobei das Loch (7), das den Mittelpunkt der radialen Anordnung (3) darstellt, geeignet ist, um für die Durchleitung der Exkrete, der Sammlung oder des Materials in eine Öffnung (7') zu expandieren, **dadurch gekennzeichnet, dass** jeder der unabhängigen Sektoren (5) mit jedem der adhäsiven Elemente (6, 26) an der Oberfläche, die der Außenseite des Beutels (2) zugewandt ist, assoziiert ist, wobei der Beutel (2) aus Polyurethan-Material aufgebaut ist und die adhäsiven Elemente (6, 9, 26) auf Polyurethan-Basis sind.

2. Der Behälter gemäß Anspruch1, **dadurch gekennzeichnet, dass** er einen Rand (8) umfasst, der ausgebildet ist, um die radialen Anordnungen (3) zu umgeben, und der mit einem entsprechenden adhäsiven Element (9) assoziiert und geeignet ist, um an der Haut des Patienten, die in Bezug auf die Öffnung peripher liegt, zu kleben.

3. Der Behälter gemäß einem vorstehenden Anspruch oder mehreren der vorstehenden Ansprüche, **dadurch gekennzeichnet, dass** er zumindest einen Schutzfilm (10, 11) aufweist, der lösbar mit dem adhäsiven Element (6, 9) des Sektors (5) und/oder dem Rand (8) in Verbindung steht.

4. Der Behälter gemäß einem vorstehenden Anspruch oder mehreren der vorstehenden Ansprüche, **dadurch gekennzeichnet, dass** er eine Lasche (12) aufweist, die eine Seite besitzt, die fest mit einem Teil des adhäsiven Elements (6, 9) des Sektors (5) und/oder des Randes (8) in Verbindung steht, und die eine gegenüberliegende Seite aufweist, die aus nichtklebendem Material gebildet ist und dazu dient, um einen Teil der Haut des Patienten daran zu hindern, an dem adhäsiven Element (6, 9) festzukleben.

5. Der Behälter gemäß einem vorstehenden Anspruch oder mehreren der vorstehenden Ansprüche, **dadurch gekennzeichnet, dass** die Sektoren (5) eine Höhe haben, die zwischen 0,5 cm und 7 cm variieren können und die bevorzugt 3,5 cm beträgt.

6. Der Behälter gemäß einem vorstehenden Anspruch oder mehreren der vorstehenden Ansprüche, **dadurch gekennzeichnet, dass** er zumindest eine Leitung (15) als Zugang zum Beutel (2) umfasst, bei denen ein Ende mit dem Beutel (2) in Verbindung steht und bei dem das gegenüberliegende Ende mit einem entfernbaren Verschlussmittel verbunden ist.

7. Der Behälter gemäß Anspruch 6, **dadurch gekennzeichnet, dass** er ein Einwegventil aufweist, das mit der Leitung (15) verbunden ist.

8. Der Behälter gemäß einem vorstehenden Anspruch oder mehreren der vorstehenden Ansprüche, **dadurch gekennzeichnet, dass** er ein röhrenförmiges Element (17) aufweist, bei dem ein Ende mit dem Beutel (2) in Verbindung steht und bei dem das gegenüberliegende Ende dazu geeignet ist, mit der Öffnung der Harnöhre verbunden zu werden und ein Mittel (18) zum Kleben an den Penis oder an die Vulva umfasst.

9. Der Behälter nach Anspruch 8, **dadurch gekennzeichnet, dass** das Klebemittel (18) ein Klebeband (19) umfasst, das auf einer Seite mit einem Klebe-Element (20) versehen und ausgebildet ist, um um den Penis geschlungen zu werden.

10. Der Behälter nach einem der Ansprüche 7 - 9, **dadurch gekennzeichnet, dass** das gegenüberliegende Ende des röhrenförmigen Elements (17) mit einem Sammelnapf (22) verbunden ist, der zum Platzieren mit Kontakt zur Vulva ausgebildet ist, wobei das Klebemittel (18) ein Klebeelement (23) umfasst, das entlang eines oberen, umlaufenden Randes (24) des Napfes (22) verteilt ist.

11. Der Behälter gemäß Anspruch 10, **dadurch gekennzeichnet, dass** ein er schwammartiges Element umfasst, das in dem Napf (22) angeordnet ist.

12. Der Behälter gemäß einem vorstehenden Anspruch oder mehreren der vorstehenden Ansprüche, **dadurch gekennzeichnet, dass** er einen schützenden Film (10, 11, 21) umfasst, der lösbar mit dem Klebeelement (6, 9) des Klebebandes (19) oder dem Rand (8) befestigt ist.

13. Der Behälter gemäß einem vorstehenden Anspruch oder mehreren der vorstehenden Ansprüche, **dadurch gekennzeichnet, dass** der Beutel zumindest zwei separate Kompartimente (51, 51) aufweist, wobei eines der Kompartimente (51) dazu vorgesehen ist, um die Fäkalien zu sammeln, wobei die radiale Anordnung (3) der Schlitze (4) dort ausgebildet ist, wobei dessen Loch (7) geeignet ist, um beim Anus (1) angeordnet zu werden, wobei das andere Kompartiment (53) angeordnet ist, um Urin zu sammeln und wobei das röhrenförmige Element (17) dort befestigt ist.

14. Der Behälter gemäß einem vorstehenden Anspruch oder mehreren der vorstehenden Ansprüche, **dadurch gekennzeichnet, dass** er Absorber und/oder koagulierende Stoffe und/oder Verdickungsmittel enthält, die in dem Beutel (2) angedeutet sind.

15. Der Behälter gemäß einem vorstehenden Anspruch oder mehreren der vorstehenden Ansprüche, **dadurch gekennzeichnet, dass** er zumindest einen auf beiden Seiten klebenden Streifen (26) umfasst, der dazu geeignet ist, um zwischen den Sektoren (5) und/oder dem Rand (8) und der Haut oder zwischen den Klebemitteln (18) und dem Penis oder der Vulva angeordnet zu werden.

16. Der Behälter gemäß Anspruch 15, **dadurch gekennzeichnet, dass** der Streifen (26) einen Schutzfilm (27) aufweist, der lösbar mit beiden seiner gegenüberliegenden Seiten (26a, 26b) verbunden ist.

## Revendications

1. Récipient pour collecter des excrétions, drainer-des collectes, purger des stomies ou similaires, comprenant un sachet (2) qui est fermé le long de ses bords périphériques et a sur au moins l'une de ses faces, un agencement radial (3) de fentes (4) qui forment une pluralité de secteurs indépendants (5), des éléments adhésifs (6, 9, 26) étant en outre prévus pour adhérer sur une partie de la peau d'un patient qui entoure l'orifice de sortie de l'excrétion, la collecte ou la matière à purger, le trou (7) qui constitue le centre dudit agencement radial (3), étant approprié pour s'expanser dans une ouverture (7') pour le passage de ladite excrétion, collecte ou matière, **caractérisé en ce que** chacun desdits secteurs indépendants (5) est associé avec chacun desdits éléments adhésifs (6, 26) au niveau de sa surface dirigée vers l'extérieur du sachet (2), le sachet (2) étant réalisé en un matériau polyuréthane et les éléments adhésifs (6, 9, 26) étant à base de polyuréthane.

2. Récipient selon la revendication 1, **caractérisé en ce qu'**il comprend un bord (8) qui est formé afin d'entourer ledit agencement radial (3) et est associé à un élément adhésif (9) respectif et est approprié pour adhérer sur la peau dudit patient qui est placée de manière périphérique par rapport audit orifice.

3. Récipient selon une ou plusieurs des revendications précédentes, **caractérisé en ce qu'**il comprend au moins un film protecteur (10, 11) qui est associé de manière détachable à l'élément adhésif (6, 9) desdits secteurs (5) et/ou dudit bord (8).

4. Récipient selon une ou plusieurs des revendications précédentes, **caractérisé en ce qu'**il comprend un rabat (12) comportant un côté qui est associé de manière rigide à une partie de l'élément adhésif (6, 9) desdits secteurs (5) et/ou dudit bord (8) et un côté opposé réalisé en un matériau non adhésif et prévu pour préserver une partie de la peau dudit patient d'une liaison adhésive avec ledit élément adhésif (6, 9).

5. Récipient selon une ou plusieurs des revendications précédentes, **caractérisé en ce que** lesdits secteurs (5) ont une hauteur qui peut varier entre 0,5 et 7 cm, de préférence égale à 3,5 cm.

6. Récipient selon une ou plusieurs des revendications précédentes, **caractérisé en ce qu'**il comprend au moins un conduit (15) pour avoir accès audit sachet (2), dont une extrémité est associée audit sachet (2) et l'extrémité opposée est associée avec des moyens de fermeture amovibles.

7. Récipient selon la revendication 6, **caractérisé en ce qu'**il comprend une soupape unidirectionnelle associée audit conduit (15).

8. Récipient selon une ou plusieurs des revendications précédentes, **caractérisé en ce qu'**il comprend un élément tubulaire (17) dont une extrémité est associée audit sachet (2) et l'extrémité opposée est appropriée pour être associée à l'orifice urétral et comporte des moyens (18) pour adhérer au pénis ou à la vulve.

9. Récipient selon la revendication 8, **caractérisé en ce que** lesdits moyens d'adhésion (18) comprennent une bande (19) qui comporte, d'un côté, un élément adhésif (20) et est appropriée pour être enroulée autour du pénis.

10. Récipient selon l'une quelconque des revendications 7 à 9, **caractérisé en ce que** ladite extrémité opposée de l'élément tubulaire (17) est associée à une coupelle de collecte (22), qui est prévue pour être placée contre la vulve, lesdits moyens d'adhésion (18) comprenant un élément adhésif (23) qui est réparti le long du bord périmétral supérieur (24) de ladite coupelle (22).

11. Récipient selon la revendication 10, **caractérisé en ce qu'**il comprend un élément spongieux logé à l'intérieur de ladite coupelle (22).

12. Récipient selon une ou plusieurs des revendications précédentes, **caractérisé en ce qu'**il comprend un film protecteur (10, 11, 21) qui est associé de manière détachable à l'élément adhésif (6, 9) de ladite bande (19) ou dudit bord (8).

13. Récipient selon une ou plusieurs des revendications précédentes, **caractérisé en ce que** ledit sachet (2) comprend au moins deux compartiments séparés (51, 52), l'un desdits compartiments (51) étant prévu pour collecter les selles, ledit agencement radial (3) de fentes (4) étant formé au niveau de celui-ci, son trou (7) étant approprié pour être agencé au niveau de l'anus (1), l'autre compartiment (53) étant prévu pour collecter l'urine et ledit élément tubulaire (17) y étant associé.

14. Récipient selon une ou plusieurs des revendications précédentes, **caractérisé en ce qu'**il comprend une matière adsorbante et/ou coagulante et/ou épaississante longée à l'intérieur dudit sachet (2).

15. Récipient selon une ou plusieurs des revendications précédentes, **caractérisé en ce qu'**il comprend au moins une languette adhésive double face (26) qui est appropriée pour être intercalée entre lesdits secteurs (5) et/ou ledit bord (8) et ladite peau ou bien entre lesdits moyens d'adhésion (18) et ledit pénis ou ladite vulve.

16. Récipient selon la revendication 15, **caractérisé en ce que** ladite languette (26) comprend un film protecteur (27) qui est associé de manière détachable à chacun de ses côtés opposés (26a, 26b).
